(19) Europäisches Patentamt
European Patent Office
Office européen des brevets

(11) Publication number: 0 244 526 A1

(12) # EUROPEAN PATENT APPLICATION

(21) Application number: 86303552.3

(22) Date of filing: 09.05.86

(51) Int. Cl.[4]: C07C 59/68 , C07C 51/295 , C07C 59/70 , A61K 31/19

(43) Date of publication of application: 11.11.87 Bulletin 87/46

(84) Designated Contracting States: CH DE FR GB LI

(71) Applicant: Council of Scientific and Industrial Research
Rafi Marg
New Delhi 110 001(IN)

(72) Inventor: Ramalingham, Thallapalli
Regional Research Laboratory Hyderabad 500 009
Andhra Pradesh(IN)
Inventor: Nageswar, Yadavalli Venkata Durga
Regional Research Laboratory Hyderabad 500 009
Andhra Pradesh(IN)
Inventor: Rao, Maddamsetty Venkateswara
Regional Research Laboratory Hyderabad 500 009
Andhra Pradesh(IN)
Inventor: Sattur, Pralhad Balvantrao
Regional Research Laboratory Hyderabad 500 009
Andhra Pradesh(IN)
Inventor: Thyagarajan, Gopalakrishna
Regional Research Laboratory Hyderabad 500 009
Andhra Pradesh(IN)

(74) Representative: Collier, Jeremy Austin Grey et al
J.A.Kemp & Co. 14, South Square Gray's Inn
London WC1R 5EU(GB)

(54) **Novel hypolipidemic and hypocholesteremic compounds and their preparation and use.**

(57) α-(3-Pentadecylphenoxy)-isobutyric acid, optionally substituted by halogen in the 4-position, and its alkali and alkaline earth metal salts, in particular the calcium salt, are useful hypolipidemic and hypocholesteremic agents or intermediates for making such agents.

EP 0 244 526 A1

## NOVEL HYPOLIPIDEMIC AND HYPOCHOLESTEREMIC COMPOUNDS AND THEIR PREPARATION AND USE

The present invention relates to therapeutic compounds useful as hypolipidemic and hypocholesteremic agents and to the preparation of such compounds.

The present invention provides substituted α-(3-pentadecylphenoxy)-isobutyric acids and their salts of the formula:

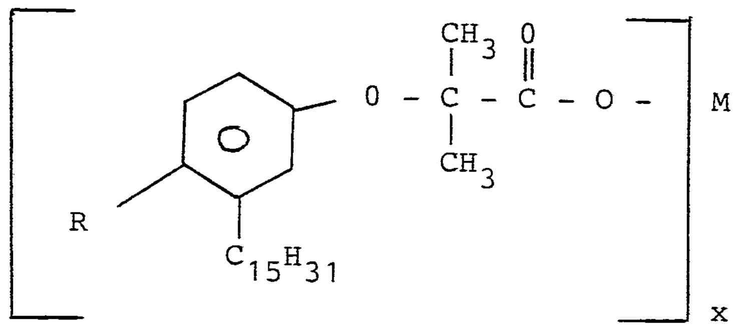

I

wherein R is hydrogen or halogen, M is hydrogen, or an alkali or alkaline earth metal, and x is 1 or 2 depending on the nature of M.

In the case of the alkali metal, e.g. sodium, salts, x is 1. In the case of the alkaline earth metal, e.g. calcium and magnesium, salts, x is 2. The preferred halogen substituent R is chlorine, and M is preferably sodium, magnesium or calcium.

The compounds of the present invention carry an alkyl chain of 15 carbon atoms in the meta position to the side chain. They have been found to possess particularly beneficial therapeutic activity in the reduction of serum cholesterol and triglyceride levels in rodents and show low toxicity. The calcium salt, for which the free acid and the sodium salt are useful intermediates, is especially active.

The α-(3-pentadecylphenoxy)-isobutyric acid of formula:

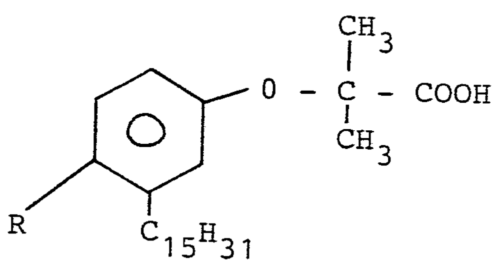

II

where R is as hereinbefore defined, is prepared, according to a feature of the invention, by reaction of a 3-pentadecylphenol of formula:

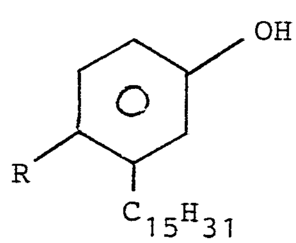

III

where R is as hereinbefore defined, with acetone and chloroform in the presence of alkali. e.g. sodium hydroxide. The reaction may be effected in excess acetone as solvent at the reflux temperature. The salt obtained may be converted, if desired, into the free acid by the action of an acid such as hydrochloric acid.

According to a further feature of the invention, the novel metal salts of formula I in which M is an alkali or alkaline earth metal are prepared by reacting α-(3-pentadecylphenoxy)-isobutyric acid of the formula II or a water-soluble salt thereof with an alkali or alkaline earth metal derivative followed by recovery of the desired product. The process may be operated, for example, by reaction of the free acid with a base such

as an alkali metal hydroxide, or an easily decomposed salt such as the carbonate. Preferably, however, the process is operated by reaction of a water-soluble salt of the free acid, e.g. the sodium salt, with a water-soluble salt, e.g. the halide, of an alkaline earth metal which forms an insoluble salt with the acid. Preferably, the reaction is carried out in an aqueous alcoholic medium wherein the alcohol is an aliphatic alcohol of 1 to 5 carbon atoms. The pH of the reaction mixture is conveniently maintained at about 8.

The calcium salt of α-(3-pentadecylphenoxy)-isobutyric acid has been investigated in detail. It possesses significant cholesterol and triglyceride reducing action in experimental animals. The sodium and magnesium salts have been found to possess substantially lower cholesterol and triglyceride reducing action.

The following Examples illustrate the invention.

## EXAMPLE 1

α-(3-pentadecylphenoxy)isobutyric acid

40 g. 3-pentadecylphenol, 200 g. acetone and 72 g. sodium hydroxide were mixed and refluxed on a steam bath for 10 minutes. 48 g. chloroform were added gradually over 30 minutes. The mixture was then refluxed for 4 hours on a steam bath. At the end of the heating, excess acetone was distilled off and the residue was dissolved in water, cooled and acidified with hydrochloric acid. The crude product was filtered and washed with water. The pure product, m.p. 55-56°C, was isolated by crystallising from cold n-hexane.

## EXAMPLE 2

Sodium α-(3-pentadecylphenoxy)-isobutyrate

100 g α-(3-pentadecylphenoxy)-isobutyric acid were dissolved in 250 ml ethanol. To this ethanolic solution, 15g sodium hydroxide were added in small portions and the entire reaction mixture was refluxed with stirring for about 30 minutes. The solvent was recovered and the residue was washed with acetone and dried. The yield of the recovered product sodium α-(3-pentadecylphenoxy)-isobutyrate was 95%, and the product had a melting point of 89° - 91°C.

## EXAMPLE 3

Calcium α-(3-pentadecylphenoxy)-isobutyrate

100 g α-(3-pentadecylphenoxy)-isobutyric acid were dissolved in 250ml ethanol. To this solution, 10g aqueous sodium hydroxide in 60ml water were added until the pH of the reaction mixture was about 8.0. A solution of 35g calcium chloride in 300ml water was added dropwise with stirring and the separated white solid was filtered, washed with water and dried.

The yield of the recovered product calcium α-(3-pentadecylphenoxy)-isobutyrate was 98%, and the product had a melting point of 106°C to 108°C.

## EXAMPLE 4

Magnesium α-(3-pentadecylphenoxy)-isobutyrate

100 g α-(3-pentadecylphenoxy)-isobutyric acid were dissolved in 250ml ethanol. To this solution, 10g aqueous sodium hydroxide in 60ml water were added until the pH of the reaction mixture was 8.0. A solution of 65g magnesium sulphate in 500 ml water was added dropwise with stirring and the gummy white solid which separated out was filtered, washed with water and dried. The yield of the gummy product identified as magnesium α-(3-pentadecylphenoxy)-isobutyrate was 94%. It could not be purified.

In the further Examples which follow, the hypolipidemic activity of different doses of the calcium salt of α-(3-pentadecylphenoxy)-isobutyric acid when administered over varying periods of time is compared with the activity of a known hypolipidemic agent, viz. clofibrate, administered over similar periods.

EXAMPLE 5

HYPOLIPIDEMIC ACTIVITY OF CALCIUM SALT OF α-(3-PENTADECYLPHENOXY)-ISOBUTYRIC ACID AND CLOFIBRATE

4 DAYS TREATMENT

Rats of both sexes were divided into three groups. Group I served as control and was treated with vehicle, i.e. gum acacia or carboxymethyl cellulose, (1ml/100g body weight). Group II was treated with the calcium salt of α-(3-pentadecylphenoxy)-isobutyric acid (200mg/kg body weight). Group III was treated with clofibrate (200mg/kg body weight). On the fifth day, all the rats were sacrificed. Blood was collected by cardiac puncture under light ether anaesthesia and the lipid profile of the serum was studied. The results are expressed as mean ± SE in Table I hereafter:

TABLE I

| LIPID PROFILE | GROUP I (n = 7) | GROUP II (n = 8) | GROUP III (n = 6) |
|---|---|---|---|
| Triglycerides mg % | 65.71 ± 2.02 | 56.25*** ± 2.23 | 43.33**** ± 1.67 |
| Serum Cholesterol Total mg % | 107.14 ± 1.84 | 75.62**** ± 2.20 | 72.50**** ± 2.14 |
| Serum Cholesterol Esterified mg % | 63.57 ± 2.25 | 59.37* ± 1.48 | 60.00* ± 1.83 |
| Serum Cholesterol Free mg % | 42.14 ± 2.14 | 16.25**** ± 1.83 | 12.50**** ± 1.12 |
| Total lipid mg % | 370.71 ± 4.24 | 356.87** ± 2.49 | 290.17**** ± 6.40 |
| Free fatty acids mEq/lit | 1.01 ± 0.02 | 0.93 ± 0.01 | 0.87**** ± 0.01 |
| Serum lipoproteins Alpha % | 39.86 ± 0.18 | 35.00** ± 1.74 | 38.67*** ± 0.18 |
| Serum lipoproteins Beta % | 60.14 ± 0.18 | 65.00** ± 1.74 | 60.14* ± 0.18 |

Significance :- Compared with control values
p values :- *ns ** <0.05 ***<0.01 ****<0.001
n :- Number of animals used.

EXAMPLE 6

HYPOLIPIDEMIC ACTIVITY OF CALCIUM SALT OF α-(3-PENTADECYLPHENOXY)-ISOBUTYRIC ACID AND CLOFIBRATE

7 DAYS TREATMENT

Rats of either sex were divided into 3 groups. Group I served as control and were treated with vehicle (1ml/100g body weight). Group II was treated with calcium salt of α-(3-pentadecylphenoxy)-isobutyric acid (200mg/kg p.o.). Group III was treated with clofibrate (200 mg/kg body weight). On the eight day, the rats were sacrificed and blood was collected by cardiac puncture under light ether aneasthesia. The lipid profile was studied and the results are expressed as mean ± SE in Table II hereafter:

TABLE II

| LIPID PROFILE | GROUP I (n = 7) | GROUP II (n = 6) | GROUP III (n = 6) |
|---|---|---|---|
| Triglycerides mg % | 65.71 ± 2.97 | 28.33**** ± 2.79 | 30.83**** ± 1.54 |
| Serum Cholesterol | | | |
| Total | 105.00 ± 2.44 | 75.00**** ± 2.58 | 80.83**** ± 2.39 |
| Esterified | 63.57 ± 1.43 | 57.50* ± 2.39 | 65.83* ± 1.54 |
| Free | 41.43 ± 2.10 | 17.50**** ± 2.01 | 15.00**** ± 1.83 |
| Total lipid mg % | 389.28 ± 11.63 | 301.33**** ± 5.07 | 342.83**** ± 8.70 |
| Free fatty acid mEq/lit | 1.027 ± 0.03 | 0.89**** ± 0.02 | 0.82**** ± 0.01 |
| Serum lipoproteins | | | |
| Alpha % | 40.00 ± 0.17 | 39.33* ± 2.56 | 39.84* ± 0.31 |
| Beta % | 60.00 ± 0.17 | 62.67* ± 2.56 | 60.16* ± 0.31 |

Significance :- Compared with control values
p values :- * ns ** $<0.05$ *** $<0.01$ **** $<0.001$
n :- Number of animals used

EXAMPLE 7

HYPOLIPIDEMIC ACTIVITY OF DIFFERENT DOSES OF CALCIUM SALT OF α-(3-PENTADECYLPHENOXY)-ISOBUTYRIC ACID

7 DAYS TREATMENT

Rats of either sex were divided into four groups. Group I served as control and were treated with vehicle (1ml/100g body weight). Groups II, III and IV were treated with calcium salt of α-(3-pentadecylphenoxy)-isobutyric acid with doses of 100 mg/kg, 200 mg/kg and 400 mg/kg orally, respectively. On the eight day all the rats were sacrificed and blood was collected by cardiac puncture under light ether anaesthesia. The lipid profile was studied and the results are expressed as mean ± SE in Table III hereafter:

TABLE III

| LIPID PROFILE | GROUP I (n = 7) | GROUP II (n = 6) | GROUP III ( n = 6) | GROUP IV (n = 6) |
|---|---|---|---|---|
| Triglycerides mg % | 65.71 ± 2.97 | 64.16* ± 1.54 | 28.33**** ± 2.79 | 30.83**** ± 2.39 |
| Serum Cholesterol Total mg % | 105.00 ± 2.44 | 92.50** ± 2.50 | 75.00**** ± 2.58 | 65.00**** ± 1.29 |
| Serum Cholesterol Esterified mg % | 63.57 ± 1.43 | 63.34* ± 2.19 | 57.50* ± 2.39 | 57.50*** ± 1.12 |
| Serum Cholesterol Free mg % | 41.43 ± 2.10 | 29.16*** ± 1.70 | 17.50**** ± 2.01 | 7.50**** ± 1.12 |
| Total lipid mg % | 389.28 ± 11.63 | 367.33* ± 0.91 | 301.33**** ± 5.07 | 218.50**** ± 4.23 |
| Free fatty acids mEq/lit | 1.03 ± 0.03 | 1.01* ± 0.01 | 0.89**** ± 0.02 | 0.79**** ± 0.01 |
| Serum lipoproteins Alpha % | 40.00 ± 0.17 | 38.17* ± 1.70 | 39.33* ± 2.56 | 32.83**** ± 0.87 |
| Serum lipoproteins Beta % | 60.00 ± 0.17 | 61.50* ± 1.98 | 60.67* ± 2.56 | 67.17**** ± 0.87 |

Significance :- Compared with control values
p values :- * ns ** $<0.05$ *** $<0.01$ **** $<0.001$
n :- Number of animals used.

EXAMPLE 8

HYPOLIPIDEMIC ACTIVITY OF CALCIUM SALT OF α-(3-PENTADECYLPHENOXY)-ISOBUTYRIC ACID AND CLOFIBRATE

15 DAYS TREATMENT

Rats of either sex were divided into three groups. Group I served as control and were treated with vehicle (1 ml/100g body weight). Group II was treated with calcium salt of α-(3-pentadecylphenoxy)-isobutyric acid (dose 200 mg/kg p.o.). Group III was treated with clofibrate (200 mg/kg p.o.). On the sixteenth day the rats were sacrificed and blood was collected by cardiac puncture under light ether anaesthesia. The lipid profile was studied and the results are expressed as mean ± SE in Table IV hereafter:

TABLE IV

| LIPID PROFILE | GROUP I (n = 7) | GROUP II (n = 8) | GROUP III (n = 6) |
|---|---|---|---|
| Triglycerides mg % | 64.28 ± 2.97 | 21.25**** ± 2.63 | 23.33**** ± 1.05 |
| Serum Cholesterol Total mg % | 105.71 ± 2.02 | 65.62**** ± 1.99 | 62.50**** ± 1.12 |
| Serum Cholesterol Esterified mg % | 62.86 ± 1.01 | 53.12**** ± 1.88 | 53.33*** ± 2.11 |
| Serum Cholesterol Free mg % | 42.86 ± 2.41 | 11.25**** ± 1.83 | 9.17**** ± 1.54 |
| Total lipid mg % | 383.29 ± 11.11 | 251.25**** ± 6.11 | 251.67**** ± 4.01 |
| Free fatty acids mEq/lit | 1.03 ± 0.04 | 0.83**** ± 0.01 | 0.79**** ± 0.004 |
| Serum lipoproteins Alpha % | 39.00 ± 0.69 | 30.37**** ± 1.02 | 35.50**** ± 0.22 |
| Serum lipoproteins Beta % | 61.00 ± 0.69 | 69.63**** ± 1.02 | 64.50**** ± 0.22 |

Significance p values :- Compared with control values
*ns ** <0.05 ***<0.01 ****<0.001

n :- Number of animals used.

EXAMPLE 9

HYPOLIPIDEMIC ACTIVITY OF CALCIUM SALT OF α-3-PENTADECYLPHENOXY)-ISOBUTYRIC ACID AND CLOFIBRATE

20 DAYS TREATMENT

Rats of either sex were divided into three groups. Group I served as control and received 1 ml of vehicle per 100 g body weight. Group II was treated with 200 mg/kg p.o. of calcium salt of α-(3-pentadecylphenoxy)-isobutyric acid. Group III was treated with clofibrate 200 mg/kg p.o. On the thirty-first day all the rats were sacrificed and blood was collected by cardiac puncture under light ether anaesthesia. The lipid profile of serum was studied and the results are expressed as mean ± SE in Table V hereafter:

TABLE V

| LIPID PROFILE | GROUP I (n = 7) | GROUP II (n = 7) | GROUP III (n = 6) |
|---|---|---|---|
| Triglycerides mg % | 66.43 ± 2.61 | 22.86**** ± 1.01 | 21.67**** ± 1.05 |
| Serum Cholesterol Total mg % | 101.43 ± 1.80 | 60.71**** ± 2.54 | 59.17**** ± 1.54 |
| Serum Cholesterol Esterified mg % | 60.71 ± 2.54 | 50.71*** ± 1.30 | 51.67** ± 1.67 |
| Serum Cholesterol Free mg % | 42.14 ± 1.01 | 7.14**** ± 1.01 | 7.50**** ± 1.12 |
| Total lipids mg % | 372.57 ± 4.30 | 234.28**** ± 5.17 | 226.67**** ± 3.33 |
| Free fatty acids mEq/lit | 0.993 ± 0.04 | 0.76**** ± 0.01 | 0,75**** ± 0.004 |
| Serum lipoproteins Alpha % | 38.29 ± 1.78 | 28.57**** ± 1.43 | 33.17** ± 0.87 |
| Serum lipoproteins Beta % | 61.71 ± 1.78 | 71.43*** ± 1.43 | 66.83** ± 0.87 |

Significance :- Compared with control values
p values :- * ns ** <0.05 *** <0.01 **** <0.001
n :- Number of animals used.

## Claims

1. α-(3-Pentadecylphenoxy)-isobutyric acid compounds of the formula

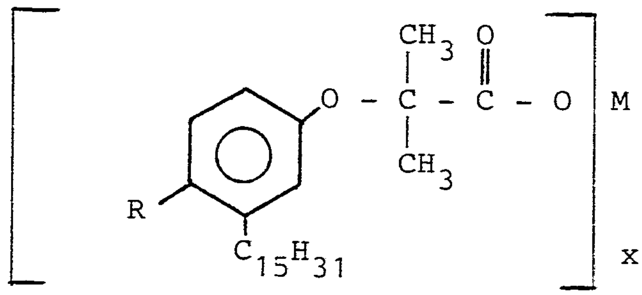

wherein R is hydrogen or halogen, M is hydrogen or an alkali or alkaline earth metal and x is 1 or, when M is an alkaline earth metal, 2.

2. α-(3-Pentadecylphenoxy)-isobutyric acid.

3. Sodium α-(3-pentadecylphenoxy)-isobutyrate.

4. Calcium α-(3-pentadecylphenoxy)-isobutyrate.

5. Magnesium α-(3-pentadecylphenoxy)-isobutyrate.

6. Process for the preparation of a α-(3-pentadecylphenoxy)-isobutyric acid or a salt thereof as claimed in claim 1 which comprises reacting 3-pentadecylphenol with acetone and chloroform in the presence of an alkali, and optionally converting the salt obtained into the free acid by the action of an acid.

7. Process for the production of a salt of α-(3-pentadecylphenoxy)-isobutyric acid as claimed in claim 1 which comprises reacting the said acid or a water-soluble salt thereof with an alkali or alkaline earth metal derivative.

8. Process as claimed in claim 7 wherein said derivative is a hydroxide, carbonate or halide or the relevant metal.

9. Process as claimed in claim 7 or 8, wherein said reaction is effected in an aqueous alcoholic medium.

10. Process as claimed in claim 7, 8 or 9, wherein the alcohol employed for said medium is an aliphatic alcohol having 1 to 5 carbon atoms.

11. Process as claimed in any of claims 7 to 10 wherein the pH of the reaction mixture is maintained at about 8.

12. A compound as claimed in claim 1 for use in therapy as a hypolipidemic or hypocholesteremic agent.

13. Calcium α-(3-pentadecylphenoxy)-isobutyrate for use in therapy as a hypolipidemic or hypocholesteremic agent.

European Patent Office

# EUROPEAN SEARCH REPORT

Application number

EP 86 30 3552

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
| --- | --- | --- | --- |
| X | CHEMICAL ABSTRACTS, vol. 92, no. 13, 31st March 1980, page 628, column 1, abstract no. 110661y, Columbus, Ohio, US; T. RAMALINGAM et al.: "alpha-(3-pentadecylaryloxy)isobutyric acids and their esters", & IN 144 918 (COUNCIL OF SCIENTIFIC AND INDUSTRIAL RESEARCH) 22-04-1978 | 1-3,6 | C 07 C 51/295<br>C 07 C 59/68<br>C 07 C 59/70<br>A 61 K 31/19 |
| | --- | | |
| A | US-A-3 262 850 (W. JONES et al.)<br>* column 6, lines 44-64, claim 1 * | 1-6,12,13 | |
| | --- | | |
| A | EP-A-0 033 980 (SCHIAPPARELLI FARMACEUTICI)<br>* claims 1, 4 * | 1,2,6,12 | TECHNICAL FIELDS SEARCHED (Int Cl 4) |
| | ----- | | C 07 C 51/00<br>C 07 C 59/00 |

The present search report has been drawn up for all claims

| Place of search | Date of completion of the search | Examiner |
| --- | --- | --- |
| BERLIN | 16-12-1986 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82